(19) **Europäisches Patentamt**
**European Patent Office**
**Office européen des brevets**

(11) **EP 1 063 012 B1**

(12) **FASCICULE DE BREVET EUROPEEN**

(45) Date de publication et mention
de la délivrance du brevet:
**11.04.2007 Bulletin 2007/15**

(51) Int Cl.:
***B01J 27/188*** *(2006.01)* ***B01J 21/06*** *(2006.01)*
***C07C 5/27*** *(2006.01)* ***C07C 2/62*** *(2006.01)*

(21) Numéro de dépôt: **00401716.6**

(22) Date de dépôt: **16.06.2000**

(54) **Nouveaux catalyseurs contenant des hétéropolyanions utilisables dans des procédés de conversion de paraffines**

Neuer heteropolyanion enthaltender Katalysator verwendbar in Paraffinumwandlungsverfahren

Novel heteropolyanion containing catalyst usable in paraffin conversion processes

(84) Etats contractants désignés:
**DE ES IT NL**

(30) Priorité: **25.06.1999 FR 9908206**

(43) Date de publication de la demande:
**27.12.2000 Bulletin 2000/52**

(73) Titulaire: **Institut Français du Pétrole**
**92852 Rueil-Malmaison Cedex (FR)**

(72) Inventeurs:
• **Travers, Christine**
**92500 Rueil Malmaison (FR)**
• **Delage, Maryline**
**92500 Rueil Malmaison (FR)**
• **Benazzi, Eric**
**78400 Chatou (FR)**
• **Joly, Jean-François**
**69006 Lyon (FR)**

(56) Documents cités:
**EP-A- 0 623 386** **EP-A- 0 864 354**
**WO-A-95/13869** **US-A- 3 374 285**

Remarques:
Le dossier contient des informations techniques présentées postérieurement au dépôt de la demande et ne figurant pas dans le présent fascicule.

EP 1 063 012 B1

**Description**

La présente invention concerne de nouveaux catalyseurs contenant des hétéropolyanions de l'acide 12-tungstophosphorique ou de l'acide 12-tungsto-molybdique et, pour certains, au moins un métal du groupe VIII, déposés sur des supports développant une surface spécifique et un volume poreux élevés, tels que l'oxyde de zirconium ($ZrO_2$), des silices, des silices-alumines ou des alumines.

Ces catalyseurs sont utilisés notamment en isomérisation des coupes paraffiniques contenant en majeure partie des n-paraffines ayant par exemple de 4 à 8 atomes de carbone par molécule et en alkylation aliphatique des isoparaffines (par exemple de l'isobutane et/ou de l'isopentane) par au moins une oléfine comprenant par exemple de 2 à 6 atomes de carbone par molécule ($C_2$ à $C_6$).

La présente invention concerne également la préparation de ces catalyseurs.

La suppression du plomb et la diminution à très court terme de la teneur en aromatiques des essences, associées à l'exigence persistante du maintien d'un indice d'octane élevé (supérieur ou égal à 95), ont conduit à rechercher des catalyseurs et des procédés améliorés permettant d'obtenir des essences à haut indice d'octane et, parmi eux, les procédés d'isomérisation des coupes paraffiniques contenant en majeure partie des n-paraffines et d'alkylation aliphatique des isoparaffines. Ces deux procédés opèrent par un mécanisme acide, mettant en jeu des carbocations comme intermédiaires réactionnels. Ils nécessitent l'utilisation de catalyseurs développant une acidité élevée.

Pour l'isomérisation des coupes paraffiniques contenant en majeure partie des n-paraffines, ces solides acides doivent conduire à une bonne activité à la plus faible température possible, l'équilibre thermodynamique favorisant les isomères multibranchés à basse température.

Les catalyseurs couramment utilisés industriellement sont :

- des catalyseurs Pt/zéolithe et plus particulièrement Pt/mordénite
- des catalyseurs à base de Pt/alumine halogénée, et plus particulièrement fortement chlorée,
- des catalyseurs à base de zircone sulfatée.

Pour l'alkylation aliphatique, ce sont les acides liquides HF et $H_2SO_4$ qui sont utilisés industriellement, malgré de gros problèmes de mise en oeuvre et de toxicité pour l'environnement.

Plus récemment, des catalyseurs contenant des hétéropolyanions ont été étudiés pour ces deux réactions. Ces hétéropolyanions sont généralement sous la forme des sels de l'acide 12-tungstophosphorique (US-A-5 482 733) ou de l'acide 12-tungstosilicique (EP-A-0 623 386 et US-A-5 391 532) échangés par de l'aluminium et déposés sur différents supports tels que $Zr(OH)_4$ ou $SiO_2$, ou encore sous la forme de l'acide 12-tungstophosphorique lui-même, supporté sur des solides mésoporeux tels que MCM-41 (US-A-5 366 945).

Dans la réaction d'isomérisation des coupes paraffiniques contenant en majeure partie des n-paraffines par exemple de 4 à 8 atomes de carbone, l'équilibre thermodynamique entre les différents isomères varie considérablement avec la température. Les hydrocarbures ramifiés, qui sont ceux qui ont un indice d'octane élevé, sont d'autant plus favorisés que la température est plus basse. Le problème de l'isomérisation de ces coupes paraffiniques consiste donc à trouver des catalyseurs actifs à la plus basse température.

En ce qui concerne les réactions d'alkylation aliphatique des isoparaffines par les oléfines, il était important de pouvoir disposer d'un catalyseur solide permettant de travailler dans les conditions les plus simples possibles et à des températures de fonctionnement supérieures à celles imposées par l'emploi des acides liquides classiques, évitant ainsi les problèmes liés au refroidissement.

C'est donc un des objets de la présente invention que de fournir de nouveaux catalyseurs améliorés dans les réactions de conversion des paraffines, aussi bien dans les réactions d'isomérisation des coupes paraffiniques contenant en majeure partie des n-paraffines que dans les réactions d'alkylation aliphatique des isoparaffines par les oléfines.

Les catalyseurs selon la présente invention sont définis d'une manière générale par le fait qu'ils comprennent au moins des hétéropolyanions dérivant de l'acide tungstophosphorique déposés sur des supports en oxyde de zirconium développant une surface spécifique et un volume poreux élevés.

Certains de ces catalyseurs comprennent en outre au moins un métal du groupe VIII. Ces derniers sont particulièrement dédiés à l'isomérisation des coupes paraffiniques contenant en majeure partie des paraffines ayant par exemple de 4 à 8 atomes de carbone. Les autres, qui ne contiennent pas de métal du groupe VIII, conviennent plus particulièrement à l'alkylation aliphatique des isoparaffines (par exemple l'isobutane et/ou l'isopentane) au moyen d'au moins une oléfine comprenant par exemple de 2 à 6 atomes de carbone par molécule ($C_2$ à C6).

Le support des catalyseurs de l'invention développe en général une surface spécifique de 50 à 500 m²/g, de préférence de 80 à 500 m²/g, et le plus souvent de 80 à 450 m²/g et un volume poreux de 0,2 à 0,9 cm³/g, de préférence de 0,3 à 0,9 cm³/g et le plus souvent de 0,3 à 0,8 cm³/g ; il est avantageusement mis sous forme de billes ou d'extrudés. La teneur en hétéropolyanion est de 10 à 55 % en poids par rapport à l'ensemble du catalyseur, de préférence de 25 à 50 % en poids.

Les catalyseurs selon l'invention particulièrement dédiés à l'isomérisation des coupes paraffiniques contenant en majeure partie des n-paraffines comprennent, outre l'hétéropolyanion et le support, au moins un métal du groupe VIII choisi par exemple parmi le platine, le palladium, le rhodium, le nickel et le ruthénium sous une teneur de 0,05 à 10 % en poids, de manière préférée de 0,1 à 5 % en poids, et de manière plus préférée de 0,2 à 1 % en poids.

Pour la préparation des catalyseurs de l'invention, les hétéropolyanions à introduire peuvent provenir de solutions aqueuses des hétéropolyacides correspondants ou de sels de ces acides. Ils sont déposés sur les supports par toute technique d'imprégnation connue de l'homme du métier et en particulier par imprégnation à sec dans le volume poreux. Avant imprégnation, les supports sont avantageusement calcinés par exemple à une température de 200 °C à 800 °C, de préférence de 350 °C à 600 °C.

Dans le cas des catalyseurs à mettre en oeuvre en isomérisation des coupes paraffiniques contenant en majeure partie des n-paraffines, on dépose sur le support au moins un métal du groupe VIII par toute méthode connue de l'homme du métier, par exemple par imprégnation.

L'hétéropolyanion et le métal du groupe VIII peuvent être co-imprégnés sur le support à partir d'une solution mixte d'un précurseur de l'hétéropolyanion (l'hétéropolyacide ou l'un de ses sels) et d'un précurseur du métal du groupe VIII. A l'issue de la co-imprégnation le catalyseur est séché à l'étuve pendant 6 à 12 heures, à une température de 100 °C à 150 °C, puis calciné sous air pendant une durée de 0,5 à 4 heures, de préférence de 1 à 3 heures, à une température de 150 °C à 400 °C, de préférence de 180 °C à 350 °C.

L'hétéropolyanion et le métal du groupe VIII peuvent également être imprégnés consécutivement, l'hétéropolyanion étant alors préférentiellement imprégné en premier. Dans ce cas, des étapes de séchage et de calcination décrites ci-dessus sont en général mises en oeuvre après l'imprégnation de l'hétéropolyanion, puis après l'imprégnation du métal du groupe VIII.

Parmi les métaux du groupe VIII précités, on préfère le platine et le palladium ; tous les sels de ces métaux suffisamment solubles dans l'eau peuvent être utilisés comme précurseurs.

À titre de métal du groupe VIII, le platine peut également être introduit dans le catalyseur par mélange mécanique avec un catalyseur $Pt/Al_2O_3$ ou $Pt/SiO_2$ préalablement réduit.

Dans le cas des catalyseurs à mettre en oeuvre en alkylation aliphatique, qui ne contiennent pas de métal du groupe VIII, on procède au dépôt des hétéropolyanions par exemple par imprégnation comme décrit plus haut, puis directement aux étapes de séchage et calcination.

Dans tous les cas, à l'issue de la calcination, on procède en général à un traitement sous hydrogène, pendant une durée de 0,5 à 4 heures, de préférence de 1 à 3 heures, à une température de 120 °C à 600 °C, de préférence de 150 °C à 500 °C.

Dans des procédés d'isomérisation utilisant les catalyseurs selon l'invention, on traite en général des charges contenant au moins 80 % en poids, de préférence au moins 90 % en poids, de paraffines de 4 à 8 atomes de carbone. Il peut s'agir plus particulièrement de coupes C4, C5-C6, C5-C7, ou C8.

La charge et l'hydrogène sont mis en contact avec un catalyseur selon l'invention comprenant au moins un métal du groupe VIII, dans des conditions d'isomérisation. Ce contact peut s'effectuer en utilisant le catalyseur en lit fixe, en lit fluidisé ou en batch (c'est-à-dire en discontinu). La réaction d'isomérisation est généralement effectuée à une température de 100 °C à 350 °C, de préférence de 150 à 300 °C, à des pressions partielles d'$H_2$ allant de la pression atmosphérique (0,1 MPa) à 7 MPa, de préférence de 0,5 MPa à 5 MPa. La vitesse spatiale peut être de 0,1 à 20, de préférence de 1 à 10, litres d'hydrocarbures liquides par litre de catalyseur et par heure. Le rapport molaire $H_2$/charge peut varier dans de larges limites ; il est normalement de 0,8/1 à 20/1, de préférence de 0,1/1 à 10/1. L'isomérisation étant une réaction équilibrée, l'isomérisat contient encore des paraffines (n-paraffines ou paraffines monobranchées) non converties. Ces paraffines peuvent être séparées des isomères, par exemple par distillation ou par fractionnement sur tamis moléculaire, et recyclées dans l'unité d'isomérisation.

Les performances des catalyseurs sont définies par la conversion (C) du n-hexane, la sélectivité ($S_i$) en isomérisation, la sélectivité en isomères dibranchés ($S_d$) et la sélectivité en craquage ($S_c$).

$$\text{Conversion (C \%)} = \frac{(\text{masse de n} - \text{hexane entrée } - \text{ masse de n - hexane sortie) x 100}}{\text{masse de n - hexane entrée}}$$

$$\text{Sélectivité isomérisation } (S_i \text{ \%}) = \frac{\text{somme (masse des iC6) x 100}}{\text{somme masses des produits de la réaction}}$$

$$\text{Sélectivité en dibranchés } (S_d \text{ \%}) = \frac{\text{(somme masse des isomères dibranchés) x 100}}{\text{(somme masse des produits de la réaction)}}$$

$$\text{Sélectivité craquage } (S_c \text{ \%}) = \frac{\text{somme (masse de } C_1 \text{ à } C_5) \text{ x 100}}{\text{somme masses des produits de la réaction}}$$

Les catalyseurs selon la présente invention qui ne contiennent pas de métal du groupe VIII peuvent être mis en oeuvre dans des procédés permettant de réaliser dans les meilleures conditions la réaction d'alkylation d'une isoparaffine (par exemple l'isobutane et/ou l'isopentane) par au moins une oléfine, par exemple de 2 à 6 atomes de carbone. En particulier, ladite réaction étant caractérisée par une forte exothermicité (environ 83,6 kJ/mol de butène transformé si l'oléfine est le butène et si l'isoparaffine est l'isobutane), la mise en oeuvre des catalyseurs selon la présente invention permet d'obtenir une bonne homogénéité de température et de concentration en réactifs.

Dans le procédé d'alkylation d'isoparaffines utilisant les catalyseurs de l'invention, les conditions opératoires, et plus particulièrement la température et la pression, sont généralement choisies de façon que le mélange constitué par l'isoparaffine, l'(les) oléfine(s) et les produits de la réaction soit liquide. De plus, il est important que le catalyseur soit immergé dans ledit liquide de façon à assurer un bon contact liquide-solide.

Le catalyseur de l'invention est avantageusement mis en oeuvre dans la zone réactionnelle d'alkylation de l'isoparaffine (isobutane et/ou isopentane) avec au moins une oléfine comprenant de 2 à 6 atomes de carbone par molécule, en phase liquide et en mélange avec l'isoparaffine et/ou un mélange d'isoparaffines. Le catalyseur selon l'invention peut être mis en oeuvre en lit expansé, en zone réactionnelle presque parfaitement agitée ou en lit circulant ; de préférence il est mis en oeuvre dans un procédé qui utilise une phase liquide continue, le catalyseur pouvant être utilisé sous forme de suspension selon les deux mises en oeuvre préférées décrites ci-après.

Une première mise en oeuvre préférée du catalyseur de l'invention est la zone réactionnelle à mélange presque parfait, c'est-à-dire à mélange parfait ou s'en rapprochant (cuve agitée ou Grignard), utilisant au moins un moyen d'agitation, par exemple au moins une hélice, de façon à obtenir une agitation suffisante du catalyseur en suspension dans la phase liquide hydrocarbonée, laquelle comprend généralement l'isoparaffine (isobutane et/ou isopentane), au moins une oléfine, éventuellement au moins un diluant inerte (par exemple le propane et le n-butane) et les produits de la réaction d'alkylation. La charge à convertir, constituée d'isobutane et/ou d'isopentane et d'au moins une oléfine, peut être par exemple introduite sous forme liquide en au moins un point au sein de la phase liquide hydrocarbonée présente dans la zone réactionnelle.

Une deuxième mise en oeuvre préférée du catalyseur selon la présente invention en suspension dans une phase hydrocarbonée est le lit mobile à co-courant, c'est-à-dire le lit circulant. Dans cette mise en oeuvre, le catalyseur en suspension dans la phase liquide hydrocarbonée, comprenant généralement l'isoparaffine (isobutane et/ou isopentane), au moins une oléfine, éventuellement au moins un diluant inerte (par exemple le propane ou le n-butane) et les produits de la réaction d'alkylation, circule de bas en haut dans la zone réactionnelle. L'ensemble constitué par la suspension de catalyseur dans la phase hydrocarbonée circule ensuite au travers d'au moins un échangeur de chaleur et d'au moins une pompe, avant d'être de nouveau introduit à l'entrée de la zone réactionnelle. La charge à convertir, constituée d'isobutane et/ou d'isopentane et d'au moins une oléfine, est introduite soit sous forme liquide, soit sous forme gazeuse en au moins un point de la zone réactionnelle.

Dans les deux types de mises en oeuvre décrites précédemment, l'isoparaffine (isobutane et/ou isopentane) n'ayant pas été convertie ou ayant été introduite en excès par rapport à la stoechiométrie de la réaction, est généralement recyclée après séparation de l'alkylat, soit par introduction directe dans la zone réactionnelle, soit par mélange avec la charge à convertir.

Le mélange isoparaffine(s)-oléfine(s) est généralement introduit dans la zone réactionnelle à une vitesse spatiale horaire, exprimée en poids d'oléfine introduite par unité de poids du catalyseur et par heure (pph), de 0,001 à 10 h$^{-1}$, de préférence de 0,002 à 2 h$^{-1}$. Ledit mélange peut aussi être réalisé à l'intérieur de la zone réactionnelle. Dans tous les cas, le mélange ainsi constitué est, dans la zone réactionnelle, dans des conditions de pression et de température telles qu'il reste liquide sur le catalyseur.

La température de réaction est généralement de 0 °C à 300 °C, de préférence de 20 °C à 200 °C. La pression de la zone réactionnelle est généralement suffisante pour maintenir les hydrocarbures à l'état liquide dans ladite zone.

Afin de limiter les réactions secondaires, on utilise généralement un excès d'isoparaffine par rapport à l'oléfine. À titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butène(s) dans la charge est généralement de 1/1 à 100/1, de préférence de 3/1 à 50/1 et de manière souvent préférée de 5/1 à 15/1.

Lorsque la nature du catalyseur et les conditions de réaction sont judicieusement choisies (en particulier la température), le catalyseur selon l'invention permet la production de produits d'alkylation d'au moins une isoparaffine par au moins une oléfine qui sont intéressants comme carburants et constituants d'essence pour les moteurs et qui comprennent par exemple au moins 60 % moles de paraffines possédant 8 atomes de carbone par molécule et moins de 1 % moles de composés non saturés, les paraffines comprenant 8 atomes de carbone par molécule comprenant 70 à 98 % en moles de triméthyl-pentanes.

Un autre avantage du catalyseur selon la présente invention est la possibilité d'alkyler, à basse température, l'isobutane avec des mélanges d'oléfines comportant de 2 à 6 atomes de carbone par molécule, où la proportion d'oléfines comportant plus de 4 atomes de carbone par molécule est très importante.

Les exemples qui suivent précisent l'invention sans en limiter la portée.

**EXEMPLE 1 :** Catalyseur A (conforme à l'invention)

49,85 g d'acide 12-tungstophosphorique ($H_3PW_{12}O_{40}$) sont dissous dans 30 cm$^3$ d'eau. Cette solution est imprégnée dans le volume poreux de 49,85 g d'un support d'oxyde de zirconium. Le solide ainsi obtenu est ensuite séché 12 heures à l'étuve à 120 °C puis calciné 2 heures à 200 °C sous air. 0,3 % en poids de platine sont alors ajoutés par mélange mécanique sous atmosphère inerte du solide précédemment préparé, avec un catalyseur Pt/Al$_2$O$_3$ pré-réduit.

**EXEMPLE 2 :** Catalyseur B (conforme à l'invention)

49,85 g d'acide 12-tungstophosphorique ($H_3PW_{12}O_{40}$) sont ajoutés à 9,5 g d'une solution d'H$_2$PtCl$_6$ renfermant 3,15 % en poids de platine. Le volume de la solution est ensuite ajusté à 30 cm$^3$. Cette solution est ensuite imprégnée dans le volume poreux de 49,85 g d'un support d'oxyde de zirconium.

Le catalyseur ainsi obtenu est séché 12 heures à l'étuve à 120 °C, puis calciné 2 heures à 200 °C sous air avant d'être traité sous H$_2$ à 220 °C pendant 4 heures.

**EXEMPLE 3 :** Test d'isomérisation du n-hexane

Les catalyseurs A et B préparés dans les exemples 1 et 2 sont testés en isomérisation du n-hexane, à 200 °C ; H2/HC (moles) = 10 ; VVH = 2 h$^{-1}$ ; et sous une pression de 0,4 MPa. Les performances respectives de ces catalyseurs sont données dans le tableau 1 ci-après.

**TABLEAU 1**

|  | Catalyseur A | Catalyseur B |
|---|---|---|
| Conversion nC$_6$ (%) | 71,1 | 68,3 |
| Sélectivité isomérisation totale (%) | 94,7 | 98,8 |
| Sélectivité en isomères dibranchés (%) | 21,9 | 28,3 |
| Sélectivité craquage (%) | 5,3 | 1,15 |

Le catalyseur B préparé par co-imprégnation de l'hétéropolyacide et du platine présente à iso-conversion une sélectivité en isomérisation considérablement améliorée, qui s'accompagne d'un craquage très fortement diminué.

**EXEMPLE 4 :** Test d'alkylation aliphatique en continu

Le catalyseur mis en oeuvre en alkylation aliphatique diffère du catalyseur décrit dans l'exemple 1 en ce qu'il ne renferme pas de platine ; la préparation est terminée après l'étape de calcination sous air.

36 g de ce catalyseur sont introduits dans un réacteur de type Grignard parfaitement agité de 500 ml muni d'une agitation mécanique, d'une entrée permettant d'injecter la charge, et d'une sortie pour récupérer les produits formés.

Le réacteur est ensuite rempli d'isobutane liquide.

Le réacteur est alors chauffé à une température de 90 °C, et l'agitation mécanique est mise en action à une vitesse de 600 tours par minute. Puis une charge contenant 10 % de butène-2 et 90 % d'isobutène (rapport molaire $iC_4$/butène-2 = 8,7 mol/mol) est injectée en continu dans le réacteur à un débit de 30 g de charge par heure, soit un pph de 0,083 g de butène-2 par gramme de catalyseur et par heure.

La pression dans le réacteur est telle que le milieu réactionnel soit liquide à la température de la réaction.

Après 95 heures d'injection de la charge, l'alkylat ($C_5$+) obtenu a la composition reportée dans le tableau 2 présenté plus loin.

La conversion de l'oléfine est supérieure à 99,8 % et l'âge du catalyseur est alors d'environ 15,7 gramme d'alkylat $C_5$+ formé par gramme du catalyseur. On le détermine en tenant compte du fait que le rendement d'alkylation est proche de 200 % en poids par rapport à l'oléfine. En effet, les produits constituant l'alkylat $C_5$+ sont majoritairement des composés ayant un nombre d'atomes de carbone double de celui de l'oléfine de départ.

### EXEMPLE 5 :

Cet exemple diffère de l'exemple 4 essentiellement par la température de réaction et le pph. En effet, la température du réacteur est de 105 °C et le pph de 1,2 g de butène-1 par gramme de catalyseur et par heure. L'oléfine utilisée est dans ce cas du butène-1 plus du butène-2.

La composition de l'alkylat obtenue après 72 heures d'injection de la charge est reportée tableau 2 ci-après.

**TABLEAU 2**

|  | Exemple 4 | Exemple 5 |
|---|---|---|
| ($C_5$-$C_7$) /$C_5$+ | 6,8 | 8,3 |
| $C_8$ (%) | 88,9 | 80,4 |
| $C_9$+ (%) | 4,3 | 11,3 |
| TMP*/$C_8$ | 89,1 | 83,8 |
| *TMP = triméthyl-pentanes | | |

### Revendications

1. Un catalyseur comprenant des hétéropolyanions de l'acide 12-tungstophosphorique, déposé sur un support en oxyde de zirconium ($ZrO_2$), **caractérisé en ce que** le support développe une surface spécifique de 50 à 500 $m^2$/g et un volume poreux de 0,2 à 0,9 $cm^3$/g.

2. Catalyseur selon la revendication 1, **caractérisé en ce que** le support développe une surface spécifique de 80 à 500 $m^2$/g et un volume poreux de 0,3 à 0,9 $c_m3$/g.

3. Catalyseur selon l'une des revendications 1 ou 2, **caractérisé en ce que** la teneur en hétéropolyanion est de 10 à 55 % en poids par rapport à l'ensemble du catalyseur.

4. Catalyseur selon l'une des revendications 1 à 3, **caractérisé en ce qu'**il comprend en outre au moins un métal du groupe VIII.

5. Catalyseur selon la revendication 4, **caractérisé en ce que** le métal du groupe VIII est choisi parmi le platine, le palladium, le rhodium, le nickel et le ruthénium et se trouve présent dans le catalyseur sous une teneur de 0,05 à 10 % en poids.

6. Procédé de préparation d'un catalyseur selon l'une des revendications 1 à 3, **caractérisé en ce que** l'on dépose sur le support des hétéropolyanions de l'acide 12-tungstophosphorique

7. Procédé de préparation d'un catalyseur selon l'une des revendications 4 et 5, **caractérisé en ce que** l'on dépose sur le support des hétéropolyanions de l'acide 12-tungstophosphorique ainsi qu'un précurseur du métal du groupe VIII.

8. Utilisation d'un catalyseur selon l'une des revendications 4 et 5 ou préparé par un procédé selon la revendication 7, en isomérisation des coupes paraffiniques contenant en majeure partie des paraffines ayant de 4 à 8 atomes de carbone par molécule.

9. Utilisation selon la revendication 8 dans laquelle la coupe paraffinique est choisie parmi les coupes C4, C5-C6, C5-C7 et C8.

10. Utilisation d'un catalyseur selon l'une des revendications 1 à 5 ou préparé par un procédé selon la revendication 6 en alkylation aliphatique d'au moins une isoparaffine par au moins une oléfine comprenant de 2 à 6 atomes de carbone par molécule.

11. Utilisation selon la revendication 10 dans laquelle l'isoparaffine est choisie parmi l'isobutane et l'isopentane.

## Claims

1. A catalyst that comprises heteropolyanions of 12-tungstophosphoric acid, deposited on a zirconium oxide substrate ($ZrO_2$) wherein the substrate develops a specific surface area of 50 to 500 $m^2$/g and a pore volume of 0.2 to 0.9 $cm^3$/g.

2. Catalyst according to claim 1, wherein the substrate develops a specific surface area of 80 to 500 $m^2$/g and a pore volume of 0.3 to 0.9 $cm^3$/g.

3. Catalyst according to one of claims 1 or 2, wherein the heteropolyanion content is 10 to 55% by weight relative to the entire catalyst.

4. Catalyst according to one of claims 1 to 3, wherein it also comprises at least one metal of group VIII.

5. Catalyst according to claim 4, wherein the metal of group VIII is selected from among platinum, palladium, rhodium, nickel and ruthenium and is present in the catalyst in a content of 0.05 to 10% by weight.

6. Process for preparation of a catalyst according to one of claims 1 to 3, wherein 12-tungstophosphoric acid is deposited on the substrate of heteropolyanions.

7. Process for preparation of a catalyst according to one of claims 4 and 5, wherein 12-tungstophosphoric acid as well as a precursor of the metal of group VIII are deposited on the substrate of heteropolyanions.

8. Use of a catalyst according to one of claims 4 and 5 or prepared by a process according to claim 7, by isomerization of paraffinic fractions that contain in large part paraffins that have 4 to 8 carbon atoms per molecule.

9. Use according to claim 8, wherein the paraffinic fraction is selected from among the C4, C5-C6, C5-C7 and C8 fractions.

10. Use of a catalyst according to one of claims 1 to 5 or prepared by a process according to claim 6 by aliphatic alkylation of at least one isoparaffin by at least one olefin that comprises 2 to 6 carbon atoms per molecule.

11. Use according to claim 10 wherein the isoparaffin is selected from among isobutane and isopentane.

## Patentansprüche

1. Katalysator, der Heteropolyanione der 12-Wolframatophosphorsäure umfasst, die auf einem Träger aus Zirkonium-oxid ($ZrO_2$) angelagert sind, **dadurch gekennzeichnet, dass** der Träger eine spezifische Oberfläche von 50 bis 500 $m^2$/g und ein Porenvolumen von 0,2 bis 0,9 $cm^3$/g entwickelt.

2. Katalysator nach Anspruch 1, **dadurch gekennzeichnet, dass** der Träger eine spezifische Oberfläche von 80 bis 500 $m^2$/g und ein Porenvolumen von 0,3 bis 0,9 $cm^3$/g entwickelt.

**3.** Katalysator nach einem der Ansprüche 1 oder 2, **dadurch gekennzeichnet, dass** der Gehalt an Heteropolyanion bei 10 bis 55 Gew.-%, bezogen auf den gesamten Katalysator, liegt.

**4.** Katalysator nach einem der Ansprüche 1 bis 3, **dadurch gekennzeichnet, dass** er ferner mindestens ein Metall der Gruppe VIII umfasst.

**5.** Katalysator nach Anspruch 4, **dadurch gekennzeichnet, dass** das Metall der Gruppe VIII ausgewählt ist aus Platin, Palladium, Rhodium, Nickel und Ruthenium und im Katalysator mit einem Gehalt von 0,05 bis 10 Gew.-% vorhanden ist.

**6.** Verfahren zur Herstellung eines Katalysators nach einem der Ansprüche 1 bis 3, **dadurch gekennzeichnet, dass** auf dem Heteropolyanionträger 12-Wolframatophosphorsäure angelagert wird.

**7.** Verfahren zur Herstellung eines Katalysators nach einem der Ansprüche 4 und 5, **dadurch gekennzeichnet, dass** auf dem Heteropolyanionträger 12-Wolframatophosphorsäure sowie ein Vorläufer des Metalls der Gruppe VIII angelagert wird.

**8.** Verwendung eines Katalysators nach einem der Ansprüche 4 und 5, oder durch ein Verfahren nach Anspruch 7 hergestellt, zur Isomerisation der Paraffinschnitte, die größtenteils Paraffine enthalten, die 4 bis 8 Kohlenstoffatome je Molekül aufweisen.

**9.** Verwendung nach Anspruch 8, wobei die Paraffinschnitte ausgewählt sind aus den Schnitten C4, C5-C6, C5-C7 und C8.

**10.** Verwendung eines Katalysators nach einem der Ansprüche 1 bis 5, oder durch ein Verfahren nach Anspruch 6 hergestellt, zur aliphatischen Alkylierung mindestens eines Isoparaffins durch mindestens ein Olefin, das 2 bis 6 Kohlenstoffatome je Molekül aufweist.

**11.** Verwendung nach Anspruch 10, wobei das Isoparaffin ausgewählt ist aus Isobutan und Isopentan.